# EUROPEAN PATENT SPECIFICATION

(11) **EP 0 796 105 B1**
(45) Date of publication and mention of the grant of the patent: **17.09.2003**
(21) Application number: 95943856.5
(22) Date of filing: 04.12.1995
(51) Int. Cl.: A61K 38/16, A61K 39/10, C07K 14/235, A61P 37/02

(54) **Use of the heterodimers S2/S4 and/or S3/S4 of the B-oligomer of pertussis toxin in the treatment of autoimmune diseases.**
Verwendung der Heterodimere S2/S4 und/oder S3/S4 des B-Oligomers des Pertussis Toxins bei der Behandlung von Autoimmunkrankheiten.
Utilisation des hétérodimères S2/S4 et/ou S3/S4 de l'oligomere B de la toxine de pertussis pour le traitement de maladies auto-immunes.

(30) Priority: 04.12.1994 IL 11186694
(43) Date of publication of application: 24.09.1997
(73) Proprietor: YEDA RESEARCH AND DEVELOPMENT CO. LTD., Rehovot 76100 (IL)
(72) Inventor: BEN-NUN, Avraham, 70600 Yavne (IL)
(74) Representative: VOSSIUS & PARTNER
(86) International application number: US9516450
(87) International publication number: WO96017620

(56) References cited:
- WO-A-92/19646
- WO-A-94/07517
- JP-A- 58 059 925
- US-A- 4 845 036
- US-A- 5 453 272
- LEHMANN D ET AL: "BACTERIAL AGENTS PROTECT AGAINST AUTOIMMUNE DISEASE. I. MICE PRE-EXPOSED TO BORDETELLA PERTUSSIS OR MYCOBACTERIUM TUBERCOLOSIS ARE HIGHLY REFRACTORY TO INDUCTION OF EXPERIMENTAL AUTOIMMUNE ENCEPHALOMYELITIS" JOURNAL OF AUTOIMMUNITY, LONDON, GB, vol. 5, 1992, pages 675-690, XP000651937 ISSN: 0896-8411
- NICOSIA A ET AL: "EXPRESSION AND IMMUNOLOGICAL PROPERTIES OF THE FIVE SUBUNITS OF PERTUSSIS TOXIN" INFECTION AND IMMUNITY, AMERICAN SOCIETY FOR MICROBIOLOGY. WASHINGTON, US, vol. 55, no. 4, 1 April 1987 (1987-04-01), pages 963-967, XP000654989 ISSN: 0019-9567
- INFECTION AND IMMUNITY, Volume 59, Number 12, issued December 1991, NENCIONI et al., "Properties of the B Oligomer of Pertussis Toxin", pages 4732-4734. XP000651936
- THE JOURNAL OF BIOLOGICAL CHEMISTRY, Volume 258, Number 11, issued 10 June 1983, TAMURA et al., "A Role of the B-Oligomer Moiety of Islet-activating Protein, Pertussis Toxin, in Development of the Biological Effects on Intact Cells", pages 6756-6761. XP002956118
- EUROPEAN JOURNAL OF IMMUNOLOGY, Volume 23, issued 1993, BEN-NUN et al., "Protection Against Autoimmune Disease by Bacterial Agents. II. PPD and Pertussis Toxin as Proteins Active in Protecting Mice Against Experimental Autoimmune Encephalomyelitis", pages 689-696. XP002965156

## Description

The present invention is generally in the field of agents that may be used for the treatment of autoimmune diseases, and more particularly relates to the use of the heterodimes S2/S4 or S3/S4 subunits of the B-oligomer, or combinations thereof, for the preparation of a medicament for protection against autoimmune diseases.

The gram-negative bacterium *Borderella pertussis (B. pertussis)*, the causative agent of whooping cough, produces several virulence factors. Pertussis toxin (PT). the major virulence component of *B. pertussis,* appears to contain an important epitope that leads to the formation of antibodies capable of protecting against the disease. Therefore, PT has been extensively investigated with regard to its possible use in preparing vaccines for whooping cough (Black et al., 1988).

Pertussis toxin is a 105-kDa hexameric protein composed of five distinct non-covalently linked polypeptides designated (in the order of decreasing molecular weight) S1-S5. PT can be divided into two distinct functional units, the enzymatically active toxic A-protomer, consisting of a single polypeptide (S1), and the pentameric B-oligomer (S2, S3, two copies of S4, and S5, i.e. molar ratio 1:1:2:1). The B-oligomer is responsible for binding of the toxin to the surface of eukaryotic target cells. The two S4 polypeptides form two distinct heterodimers with S2 and S3, which are in turn held together by S5 (see review by Gierschik, 1992).

The pertussis toxin gene has been cloned and sequenced (Nicosia et al., 1986; European Patent Application EP 0232 229; Locht and Keith, 1986; US Patent 4, 883,761). The individual subunits have been subcloned and expressed in E. *coli* in non-fusion form (Burnette et al.,1988) or as fusion proteins (Nicosia et al., 1987), and tested as antigens for prgtection against whooping cough.

The development of experimental autoimmune encephalomyelitis(EAE), as well as other autoimmune diseases in experimental animals, can be facilitated by injecting *Bordetella pertussis* concomitantly with inoculation of the autoantigen (Bernard et al., 1992). EAE is a neurological autoimmune disease which can be induced in experimental animals by a single injection of central nervous system (CNS) tissue homogenate or purified myelin antigens such as myelin basic protein (MBP) or proteolipid protein (PLP) in complete Freund's adjuvant (CFA) (Tabira and Kira, 1992). The clinical and pathological features of EAE are reminiscent of multiple sclerosis , and EAE is a well-accepted model for multiple sclerosis. In mice, consistent elicitation of EAE was shown to be facilitated by administration of B. *pertussis* at the time of the encephalitogenic challenge (Munoz, 1985). Pertussis toxin (PT) was shown later to be the component of *B. pertussis* responsible for facilitating disease development, and it is now routinely used in place of B. *pertussis* for enhancement of autoimmune disease in experimental animals (Munoz, 1995).

In an analysis of the possible immunomodulating activity of various bacteria, it was found by the present inventor that *B. pertussis* not only enhances the development of EAE in mice, but can also protect against the disease depending on the time and route of injection (Lehman and Ben-Bun, 1993). The protective activity of B. *pertussis was* subsequently assigned to PT (Ben-Nun et al., 1993).

Pertussis toxin (PT), the major virulence determinant of B. pertussis, is composed of two distinct functional units: the A-protomer consisting of a single polypeptide (S1) that mediates adenosine diphosphate (ADP)-ribosylation of host G proteins, and the B-oligomer, a complex pentamer composed of subunits S2, S3, S4 and S5 in a respective molar ratio of 1:1:2:1, which mediates the binding of the toxin to host tissue by interaction with glycoproteins and glycolipids on many types of eukaryotic cells (Gierschik, 1992). PT was found to have mitogenic and immunoadjuvant properties (Munoz, 1985). The mechanism by which PT can enhance the development of EAE in mice is not yet clear. However, it has been suggested that PT facilitates the access of autoantigen-specific T cells to the CNS by affecting the vascular permeability of the blood-brain barrier (Linthicum et al., 1982).

The mechanism by which PT protects against the development of EAE is also unclear. To further understand how both enhancing and protective activities are mediated by the same, albeit complex. molecule, it is essential to delineate the regions of the PT holomer which may be associated with one or both of these activities.

It has now been found in accordance with the present invention that the heterodimer S2/S4 or S3/S4 subunits of the B-oligomer of pertussis toxin or a combination thereof, are able to block the development of EAE in mice. Since EAE is a well-established and widely accepted animal model for the study of autoimmune diseases, these findings indicate that the heterodimer S2/S4 or S3/S4 subunits of the B-oligomer of pertussis toxin or a combination thereof, will be useful for the protection against autoimmune diseases in humans.

The invention relates to the use of the heterodimer S2/S4 or S3/S4 subunits of the B-oligomer of pertussis toxin, or a combination thereof, for the manufacture of a pharmaceutical composition useful for the protection against autoimmune diseases in humans.

Any autoimmune disease can be treated with a pharmaceutical composition of the invention, such as rheumatoid arthritis, systemic lupus erythematosus, insulin-dependent diabetes mellitus and graft-versus-host disease, and more particularly, multiple sclerosis.

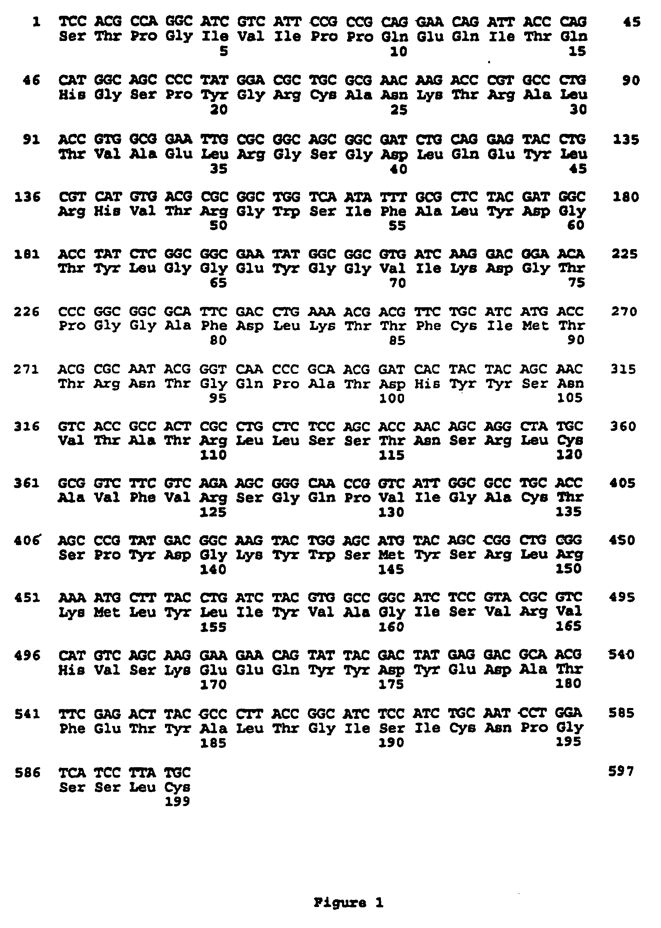

**Fig. 1** depicts the nucleocide and amino acid sequence of the subunit S2 of the B-oligomer of pertussis toxin.

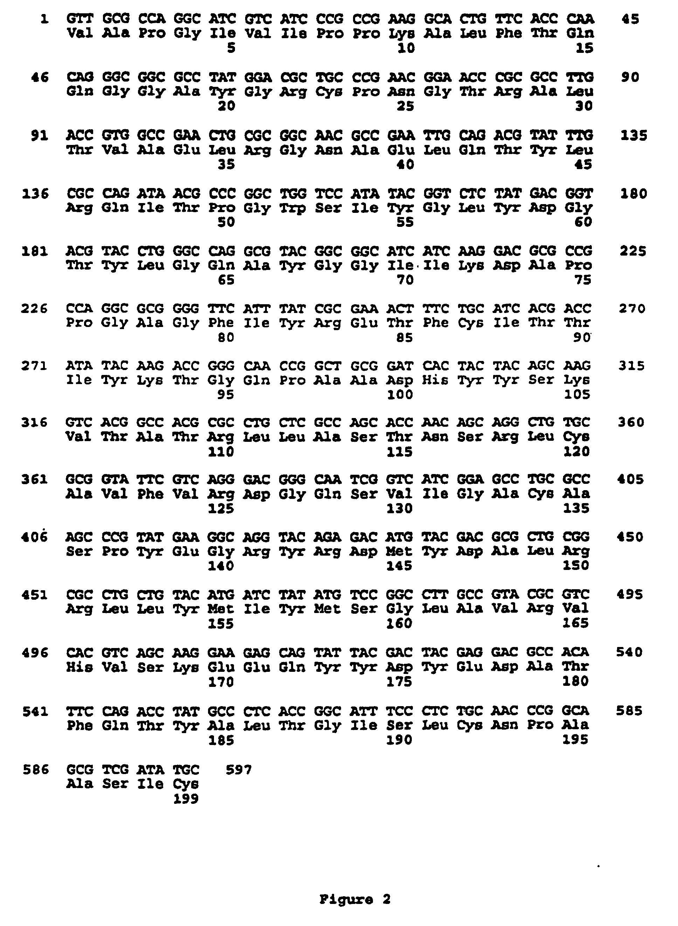

**Fig.2** depicts the nucleotide and amino acid sequence of the subunit S3 of the B-oligomer of pertussis toxin.

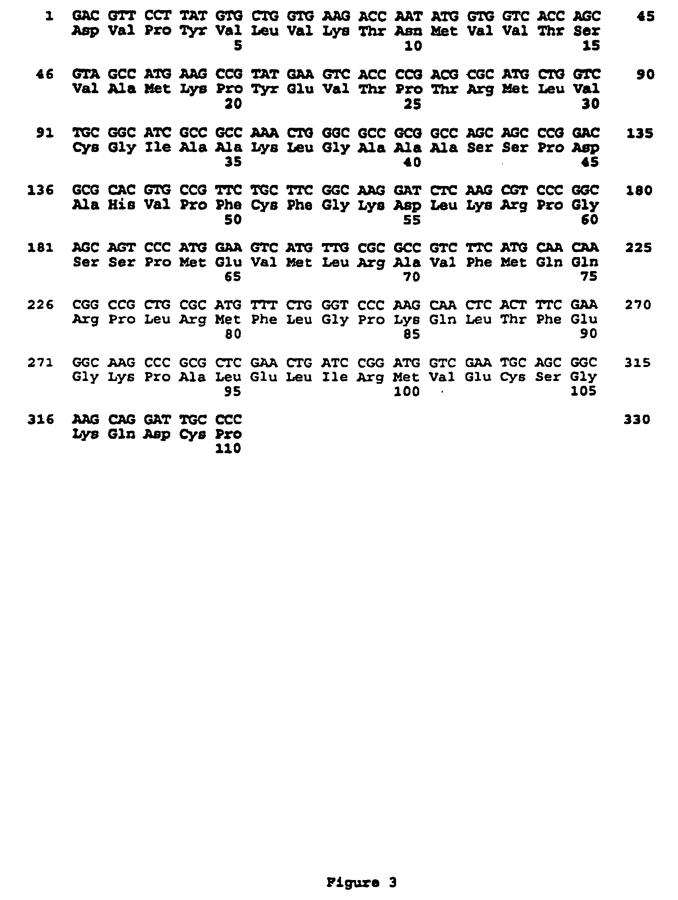

**Fig. 3** depicts the nucleotide and amino acid sequence of the subunit S4 of the B-oligomer of pertussis toxin.

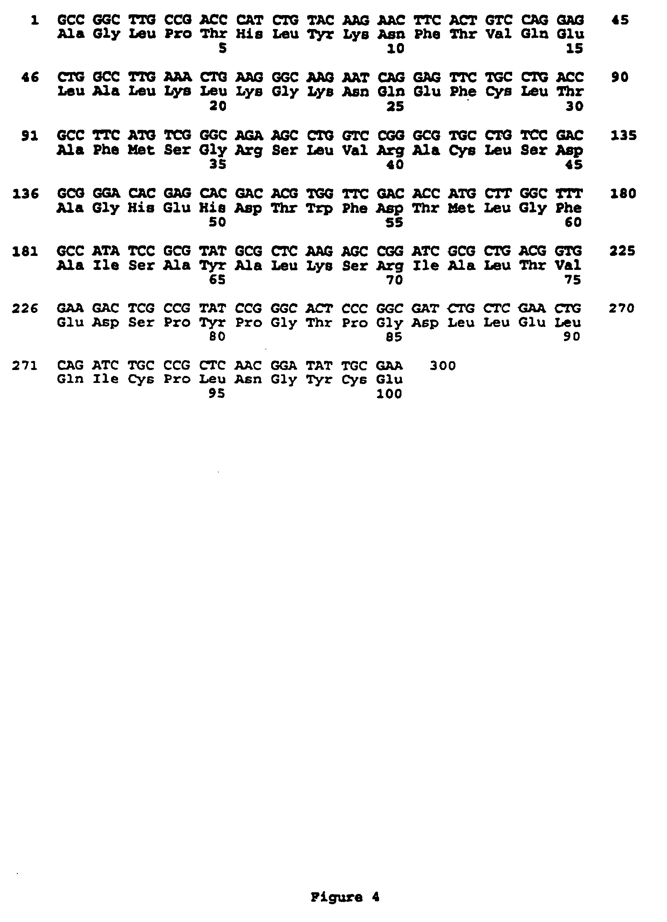

**Fig. 4** depicts the nucleotide and amino acid sequence of the subunit S5 of the B-oligomer of pertussis toxin.
**Fig. 5** shows protection against the development of EAE by PT administered in oil or in aqueous solution. PT (400ng) in 0.1 ml emulsion of IFA or in aqueous solution (PBS) was injected s.c. in the flanks, 18 days prior to the encephalitogenic challenge. X denotes mortality of all mice in the group.P and M represent incidence of paralysis and mortality, respectively, in each group. a) p = 0.0002, and b) p =0.0012 when compared to P or M, respectively, in the combined controls (None + IFA).
**Fig. 6** shows that the B-oligomer blocks the development of EAE. B-oligomer (100 ng) or PT (400 ng) emulsified in IFA were injected s.c. 16 days prior to the encephalitogenic challenge. a) p = 0.001, and b) p = 0.001 when compared to P or M, respectively, in the combined controls (None + IFA).
**Fig. 7** shows silver-stained SDS-gel. of gel-purified PT subunits. The subunits were isolated by preparative gel electrophoresis and analyzed. Five µg whole PT, 0.4µg purified S1, 0.3µg purified S2, 0.7µg purified S3 and 1.25µg purified S4/S5 were run on the relevant lanes.
**Fig. 8** shows differential protective effect of the B-oligomer subunits. 100 ng of B-oligomer or of gel-purified subunits were emulsified in IFA and injected s.c. 20 days before the encephalitogenic challenge, a) p = 0.0002, b) p = 0.0043, c) p = 0.0016, and d) p = 0.014 when compared to P in the combined controls (None + IFA); e) p = 0.001, f) p = 0.008 and g) p = 0.04 when compared to M in the combined controls (None ö IFA).
**Fig. 9** shows that the B-oligomer does not promote the development of EAE. PT (400 ng) or B-oligomer (400 ng) were injected I.V. immediately, and 48 hours after mice were injected with MSCH/CFA to induce the development of EAE. a) p = 0.004. and p = 0.02 when compared to P or M, respectively, in the +PT group.

PT administered in oil or in aqueous solution protects mice against the development of EAE (Fig. 5), which is routinely used as a model for organ-specific T-cell mediated autoimmune diseases, suggesting its potential efficacy for therapy of such diseases. However, the use of PT as a therapeutic agent is rather risky in view of its toxicity.

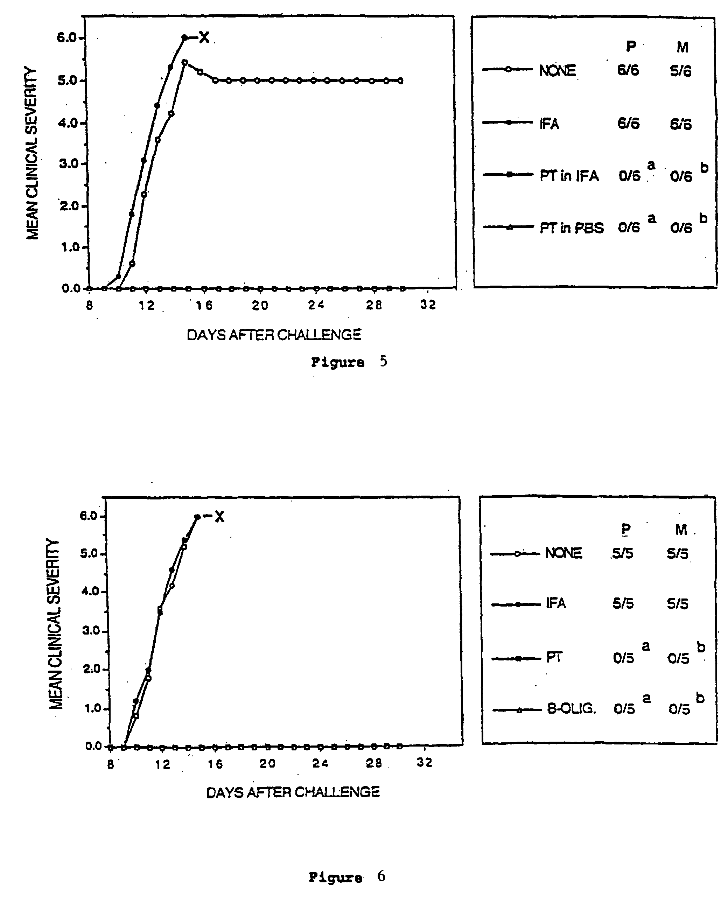

In order to identify regions of PT associated with enhancement or with blocking of disease, both for potential therapeutic use and for a better understanding of the mechanisms of action of PT on autoimmune diseases, the effect of the constitutive units of PT on the development of EAE was investigated according to the present invention. The findings shown herein in the examples that the B-oligomer of PT, which does not contain the toxic S1 monomer. has a remarkable protective effect against EAE (Fig. 6), indicate that the B-oligomer is responsible for the protective activity of PT. Furthermore, the failure of the B-oligomer to enhance the development of EAE (Fig. 9) indicates that the enhancement of autoimmune diseases by PT is likely to be associated with the S monomer of PT.

Several features of the B-oligomer of PT make it a potentially useful agent for the therapy of autoimmune diseases: i) it is highly potent in blocking the development. of EAE; ii) it is devoid of the enhancing activity of PT; and iii) the B-oligomer, which does not comprise the toxic S1 monomer, is non-toxic in vivo.

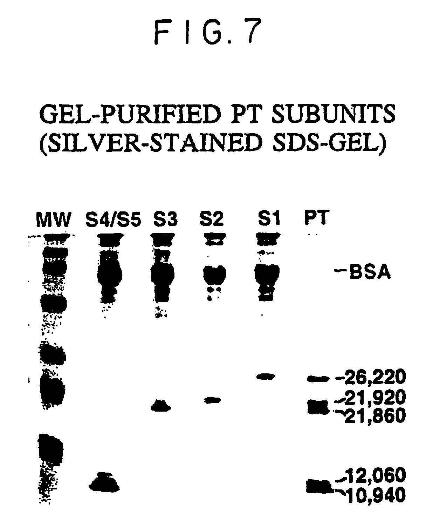

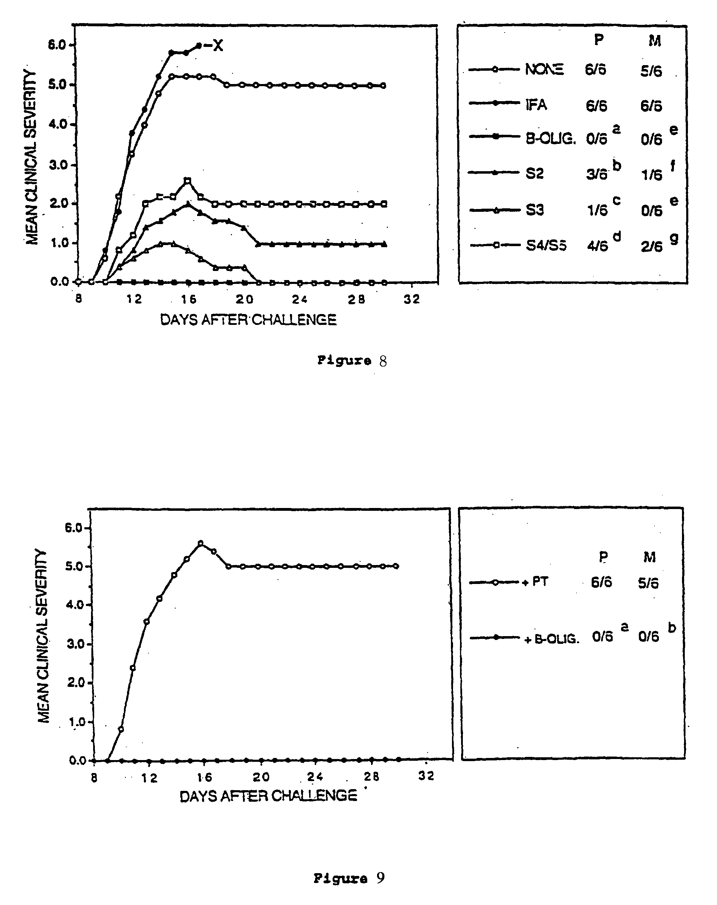

The subunits S2, S3, S4 and S5 of the B-oligomer were purified (Fig. 7) and tested for their ability to protect against EAE, in the attempt to assign the protective activity of the B-oligomer to a particular subunit. The findings that each subunit has protective activity (Fig. 8) further indicate that each can be used as a therapeutic agent for autoimmune diseases; Each subunit protected against EAE to a different extent, and the protection conferred was not as complete as that given by the B-oligomer. indicating that various combinations of the subunits in the form of mixtures, e.g. S2+S3, S2+S4, S2+S3+S5, or as heterodimers, e.g. S2/S4, S3/S4, may be more effective in abolishing disease development.

Hence, the B-oligomer of PT and the subunits of the B-oligomer, individually or in various combinations, are potentially useful agents for the therapy of multiple sclerosis and other autoimmune diseases, such as rheumatoid arthritis, insulin-dependent diabetes mellitus, systemic lupus erythematosus, and graft-versus-host disease.

Thereby, the present invention comprises as active ingredient the hererodimer S2/S4 or S3/S4 subunits, or a mixture of said heterodimers.

The B-oligomer of PT is commercially available or can be prepared by any method described in the art, such as for example by dissociation of pertussis toxin into the A protomer and the B-oligomer by affinity chromatography (Tamura et al., 1982: and 1983; Burns et al., 1987), or by combining hererodimer S2/S4, heterodimer S3/S4 and subunit S5 at the 1:1:1 molar ratio in 2M urea (Tamara et al., 1983). All attempts to prepare recombinant B-oligomer have not been successful.

The individual subunits S2, S3, S4 and S5. both native and recombinant, are encompassed by the invention. The native subunits can be prepared, for example, by preparative gel electrophoresis as described according to the present invention (Fig. 7) or by gel filtration (Tamura et al., 1982), and the recombinant subunits as described by Nicosia et al, 1987, and Burnette et al., 1988. The heterodimers S2/S4 and S3/S4 are also prepared according to Tamura et al., 1982.

The pharmaceutical compositions comprise a pharmaceutically acceptable carrier and as active ingredient the heterodimer S2/S4 and S3/S4 subunits associated similarly to the native molecule, or a mixture of said heterodimers.

The pharmaceutical compositions are prepared by mixing the active ingredient with a pharmaceutically acceptable carrier, stabilizers and excipients, and prepared in dosage form, e.g. by lyophilization in dosage vials. They may be administered in all suitable ways, e.g. intravenously, intramuscularly, subcutaneously, local injection, topical application or per os, as the case may require. The amount of active compound to be administered will depend on the route of administration, the disease to be treated and the condition of the patient.

The invention will now be described in more detail in the following non-limiting examples and their accompanying figures.

### Materials and Methods

### (a) Materials

Pertussis toxin (PT) was obtained from Sigma (St Louis, Mo, USA) and also as a kind gift from Dr. D. Teitelbaum (The Weizrnann Institute of Science, Rehovot, Israel): The B-oligomer of PT was obtained from List Biological Laboratories Inc. (Campbell, CA).

### (b) Mice

Female SJL/J mice were purchased from Jackson Laboratories ( Bar Harbor, Me, USA). All mice were 2-3 months old when used in the experiments.

### (c) Induction of EAE

EAE was induced in mice as previously described (Ben-Nun and Lando, 1983). Briefly, 0.1 ml of emulsion prepared from mouse spinal cord homogenate (MSCH, 60 mg/ml) emulsified with an equal volume of CFA enriched for M. tuberculosis H37Ra (5mg/ml) was injected s.c. into the mouse footpads. Immediately after and 48 hrs later, PT was injected intravenously (400 ng/mouse). Where stated in the text. the B-oligomer (400 ng/mouse) was also used in place of PT to evaluate its effect on disease enhancement

### (d) Clinical evaluation, scoring and statistical analysis

Following the encephalitogenic challenge, mice were observed daily for clinical signs of EAE. The severity of the clinical manifestations was scored daily for each individual mouse in the treatment group on a scale of 0-6; 0: no clinical signs, 1: loss of tail tonicity, 2: flaccid tail, 3: hind leg paralysis, 4: hind leg paralysis with hind body paresis, 5: hind and fore leg paralysis, 6: death. Results presented denote the mean score of the treatment group on each given day after encephalitogenic challenge. At the peak of the clinical disease, on days 12-17 after encephalitogenic challenge, the standard errors (not shown) were less than 16% of the mean clinical score. Each graph also displays the incidence of paralysis (P) and mortality (M) for each treatment group in order to allow a complete evaluation of the clinical course of EAE.

To evaluate the statistical significance of the differences in incidence of paralysis or mortality between treatment and control groups in each experiment, the contingency table analysis was used to calculate the χ² values with the Stat View 512⁺ program. Due to the relatively small number of mice in each group, the *p* values given are of χ² with continuity correction.

### (e) Protection against EAE

For the induction of protection against the disease, mice were injected s.c. in the flanks with 400 ng of PT, or with 100 ng of B-oligomer or gel-eluted subunits of the B-oligomer, emulsified in incomplete Freund's adjuvant (IFA). A total volume of 0.1 ml was injected 2-3 weeks prior to encephalitogenic challenge. Where indicated, PT was injected in aqueous solution (PBS) s.c. in the flanks, in a total volume of 0.1 ml.

### Example 1. Effect of pertussis toxin on the development of EAE

Fig. 5 shows that a single injection of PT prior to the encephalitogenic challenge has a dramatic effect on the subsequent development of EAE induced in SJL/J mice by CNS tissue homogenate. PT was remarkably effective in blocking the development of EAE regardless of whether it was administered s.c. as an oil emulsion or an aqueous solution. These results show that PT can not only facilitate the development of autoimmune diseases as previously shown (Munoz, 1985), but also has a strong protective activity which can completely abolish disease development (Ben-Nun et al., 1993).

### Example 2. Effect of the B-oligomer of pertussis toxin on the development of EAE

To identify regions of the PT molecule that can be associated with the enhancing effect or with the protective activity of PT, the A-protomer and the B-oligomer were investigated. Fig. 6 shows that the B-oligomer, which is devoid of the toxic S1 subunit, is sufficient to protect against the development of EAE. The protection against EAE was total with as little as 50-100 ng of B-oligomer per mouse administered as a single injection. B-oligomer was at least as potent as whole PT in protecting mice against the disease.

### Example 3. Purification of PT subunits by elution from preparative SDS-gels and their effect on the development of EAE

In the attempt to assign the protective activity of PT to a particular subunit of the B-oligomer, PT (Sigma) was electrophoresed on preparative SDS-gel according to Laemmli, 1970. Each subunit was located according to stained reference lane on each gel (Fig. 7). The unstained relevant bands were cut out from the gel. Each subunit was eluted from the crushed gel band by passive diffusion, dialysed extensively against double distilled H₂O in the presence or absence of bovine serum albumin, lyophilised and resuspended in PBS.

The B-oligomer, subunits S2, S3 and S4/S5 were tested in vivo for protective activity against the development of EAE. As shown in Fig. 8, each of the subunits had a protective effect. However, although the protective activity of the different subunits was marked, the protection was not as total as that obtained with B-oligomer or whole PT.

### Example 4. The B-oligomer does not promote development of EAE in mice

As mentioned above, PT has a conflicting immunomodulatory effect on autoimmune diseases (Ben-Nun et al., 1993). It can facilitate the development of the disease and also protect against it (as shown here). As the B-oligomer was found to be highly effective in protecting against EAE, its possible enhancing effect on the disease was investigated. Fig. 9 shows that in comparison with PT, that facilitated the development of a disease with severe neurological impairment and high mortality, the B-oligomer had no enhancing effect. Thus, all mice that received PT concomitantly with the encephalitogenic challenge developed severe EAE, whilst all mice that received PBS or B-oligomer remained healthy.

### References

1. Ben-Nun, A. and Z. Lando. 1983. Detection of cells responding to myelin basic protein by proliferation and selection of T cell lines functional in mediating experimental autoimmune encephalomyelitis (EAE) in mice. *J. Immunol.* 130:1205.
2. Ben-Nun, A., S. Yossefi and D. Lehman. 1993. Protection against autoimmune disease by bacterial agents. II. PPD and pertussis toxin as proteins active in protecting mice against experimental autoimmune encephalomyelitis. *Eur. J. Immunol.* 23:689.
3. Bernard, C.C.A, Mandel, T.E. and Mackay, I.R. 1992. Experimental models of human autoimmune diseases: Overview and prototypes. In *The autoimmune diseases II*, Rose, N. and Mackay, I.R. (eds), Academic Press, N.Y., p.47.
4. Black, W.J., Munoz, J.J., Peacock, M.J., Schad, P.A., Cowell. J.L., Burchall, J.J., Lim, M., Kent, A., Steinman, L. and Falkow, S. 1988. ADP-ribosyltransferase activity of pertussis toxin and immunomodulation by Bordetella pertussis. *Science* 240:656.
5. Burnette, W.N. et al. 1988. Direct expression of Bordetella pertussis toxin subunits to high levels in Escherichia coli. *Bio*/*Technology* 6:699-706.
6. Burns, D.L. et al. 1987. Role of the A Subunit of Pertussis Toxin in Alteration of Chinese Hamster Ovary Cell Morphology. *Infection and Immunity* 55:24-28.
7. Gierschik. P. 1992. Guanine Nucleotide-Binding Proteins by Pertussis Toxin. *Current Topics in Microbiology and Immunology,* Springer-Verlag, Berlin, 175:69-96.
8. Laemmli. U.K. 1970. Cleavage of structural proteins during the assembly of the head of bactetiophage T4. *Nature* (Lond). 227:680.
9. Lehman, D. and A. Ben-Nun. 1992. Bacterial agents protect against autoimmune disease. I. Mice pre-exposed to Bordetella pertussis or Mycobacterium tuberculosis are highly refractory to induction of experimental autoimmune encephalomyelitis. *J. Autoimmun.* 5:675.
10. Linthicum. D.S., Munoz, J.J. and Blaskett, S. 1982. Acute experimental autoimmune encephalomyelitis in mice. I Adjuvant action of Bordetella pertussis is due to vasoactive amine sensitization and increased vascular permeability of the central nervous system. C*ell Immunol.* 73:299
11. Locht, C. and Keith, J.M. 1986. Pertussis Toxin Gene: Nucleotide Sequence and Genetic Organization. *Science.* 232:1258-1264.
12. Munoz. J.J. 1985. In *Pertussis toxin,* Sekura, RD., Moss, J. and Vaughan, M. (eds), Academic Press, N.Y. p. 1.
13. Nicosia, A. et al. 1986. Cloning and sequencing of the pertussis toxin genes: Operon structure and gene duplication. *Proc. Natl. Acad Sci.* USA.83:4631-4635.
14. Nicosia, A. et al. 1987. Expression and Immunological Properties of the Five Subunits of Pertussis Toxin. *Infection and Immunity. 55:963-967.*
15. Tabira, T. and J.-I. Kira. 1992. Strain and species differences of encephalitogenic determinants of myelin basic protein and proteolipid apoprotein. In *Myelin: Biology and chemistry,* R.E. Martenson, ed. CRC Press, USA. p.783.
16. Tamura, M. et al. 1982. Subunit Structure of Islet-Activating Protein, Pertussis Toxin, in Conformity with the A-B Model. *Biochemistry.* 21:5516-5522.
17.Tamura M. et al., 1987 A Role of the B Oligomer Moiety of Islet Activating Protein, pertussis Toxin, in the Development of the Biological Effects on Intact Cells. *J. Biol. Chem.* 258:6756-6761.

## Claims

1. Use of a protein selected from the group comprising the heterodimer S2/S4 or S3/S4 subunits of the B-oligomer of pertussis toxin (PT), or a mixture thereof, for the preparation of a medicament for the treatment of autoimmune diseases.

2. Use according to claim 1 wherein the autoimmune disease is selected from the group, comprising multiple sclerosis, rheumatoid arthritis, systemic lupus erythematosus, insulin-dependent diabetes mellitus and graft-versus-host disease.

3. Use according to claim 2 wherein the autoimmune disease is multiple sclerosis.

4. Use according to any one of claims 1 to 3, wherein the protein is the heterodimer S2/S4.

5. Use according to any one of claims 1 to 3, wherein the protein is the heterodimer S3/S4.

6. Use according to any one of claims 1 to 3, wherein the protein is a mixture of the heterodimers S2/S4 and S3/S4.

## Patentansprüche

1. Verwendung eines Proteins, ausgewählt aus der Gruppe, umfassend die heterodimeren S2/S4- oder S3/S4-Untereinheiten des Pertussistoxin(PT)-B-Oligomers oder ein Gemisch davon, für die Herstellung eines Medikaments zur Behandlung von Autoimmunerkrankungen.

2. Verwendung nach Anspruch 1, wobei die Autoimmunerkrankung ausgewählt ist aus der Gruppe, umfassend multiple Sklerose, rheumatoide Arthritis, systemischer Lupus erythematodes, Insulin-abhängiger Diabetes mellitus und Graft-versus-Host-Erkrankung.

3. Verwendung nach Anspruch 2, wobei die Autoimmunerkrankung multiple Sklerose ist.

4. Verwendung nach einem der Ansprüche 1 bis 3, wobei das Protein das S2/S4-Heterodimer ist.

5. Verwendung nach einem der Ansprüche 1 bis 3, wobei das Protein das S3/S4-Heterodimer ist.

6. Verwendung nach einem der Ansprüche 1 bis 3, wobei das Protein ein Gemisch der S2/S4- und S3/S4-Heterodimere ist.

## Revendications

1. Utilisation d'une protéine sélectionnée parmi le groupe comprenant les sous-unités hétérodimèriques S2/S4 ou S3/S4 de l'oligomère B de la toxine pertussique (TP), ou un mélange de ces dernières, pour la préparation d'un médicament destiné au traitement des maladies auto-immunes.

2. Utilisation selon la revendication 1 **caractérisée en ce que** la maladie autoimmune est sélectionnée parmi le groupe comprenant la sclérose en plaque, la polyarthrite rhumatoïde, le lupus érythémateux disséminé, le diabète sucré insulino-dépendant et la maladie du greffon versus de l'hôte.

3. Utilisation selon la revendication 2 **caractérisée en ce que** la maladie autoimmune est la sclérose en plaque.

4. Utilisation selon l'une quelconque des revendications 1 à 3, **caractérisée en ce que** la protéine est l'hétérodimère S2/S4.

5. Utilisation selon l'une quelconque des revendications 1 à 3, **caractérisée en ce que** la protéine est l'hétérodimère S3/S4.

6. Utilisation selon l'une quelconque des revendications 1 à 3, **caractérisée en ce que** la protéine est un mélange des hétérodimères S2/S4 et S3/S4.
